# EUROPEAN PATENT APPLICATION

(11) **EP 1 505 348 A2**
(43) Date of publication of application: **09.02.2005**
(21) Application number: 04018767.6
(22) Date of filing: 06.08.2004
(51) Int. Cl.: F24C 1/00, F24C 15/00, A61L 9/22

(54) **Stove or ventilated fireplace with a forced air circulation system**

(30) Priority: 08.08.2003 IT vr20030101
(71) Applicant: Gruppo Piazzetta S.p.A., 31010 Casella d'Asolo (Treviso) (IT)
(72) Inventor: Piazzetta, Domenico, 31010-Casella D'Asolo Treviso (IT)
(74) Representative: Reniero, Cirillo Silvano

(57) **Abstract**

Stove or ventilated fireplace, preferably for burning wood or pellets, including a forced air circulation system (8) and one or more air ionising devices (21) for purification of room air.

## Description

The present invention relates to a stove, and more particularly to a wood or pellet stove, or a ventilated fireplace equipped with a forced air circulation system.Stoves and fireplaces equipped with forced air circulation system intended to take in air and deliver it (possibly heated) to the room in which the stove or ventilated fireplace is installed, and, possibly, to other rooms in communication with the stove or fireplace via suitable ducts, have already been suggested in the state of the art. See for instance the Italian patent application no. VR2002U000001 filed on 10.01.2002 in the name of the same applicant as that of the present application and entitled "Improved pellet stove".

Such forced air circulation systems have the advantage of forcing heated air to circulate. However, should the environmental air be stale or even polluted, also pollutants are caused to circulate which constitutes a serious prejudice for the healthiness of the environment.

The main object of the present invention is to provide an stove, and in particular a wood stove, or ventilated fireplace equipped with a forced air circulation system capable of purifying the environmental air whether the fireplace or stove is on or off.

Another object of the present invention is that the said wood stove or ventilated fireplace can be manufactured at a competitive production costs.

These and other objects that will be better apparent below are achieved by a stove or ventilated fireplace according to the present invention including a forced air circulation system, and characterised in that it comprises at least one ionising device for the air being circulated.

Further aspects and advantages of the present invention will better appear from the detailed description given below of a pellet stove and ventilated fireplace, according to the present invention, given for non limiting illustration purposes in the accompanying drawings, in which:
Figure 1 is a lateral elevation view of a pellet stove;
Figure 2 is a cross-sectional view on an enlarged scale taken along an intermediate vertical plane of the stove shown in Figure 1;
Figure 3 is a cross-sectional view taken along a vertical plane of a ventilated fireplace according to the present invention;
Figure 4 is a functional diagram of an air ionising device for a stove or fireplace according to Figures 1 to 3; and
Figure 5 shows a detail of Figure 4.

In the accompanying drawings identical or similar parts or components are marked with the same reference numerals.

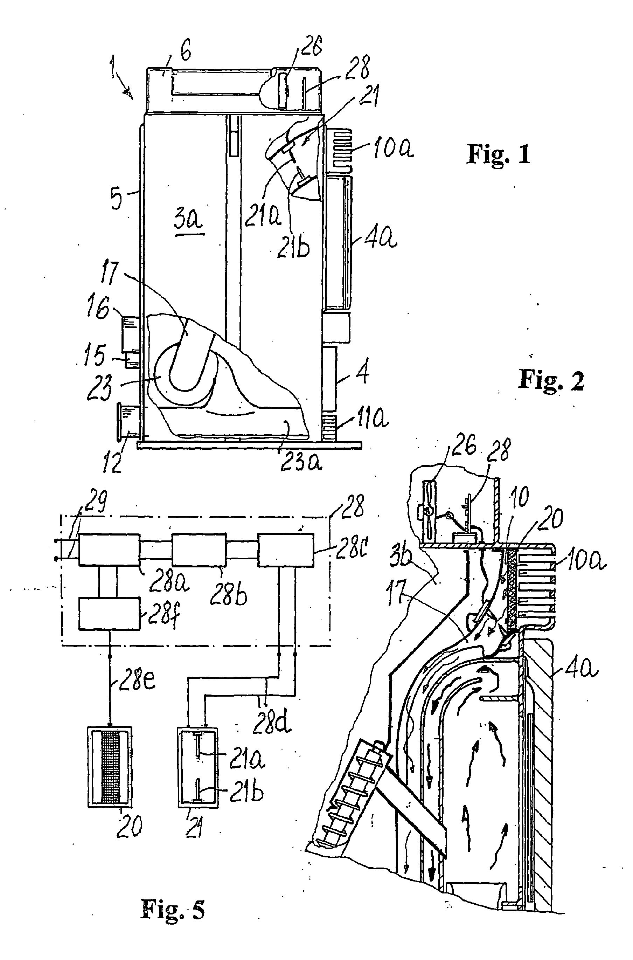

With reference to the above Figures, 1 indicates a stove and 1a a ventilated fireplace according to the present invention. Figures 1 to 3 show a stove 1 comprising a substantially parallelepiped body rectangular in a plane view, having a base 2, two side panels or walls 3a and 3b, a front wall 4 with a door 4a, a rear walll 5, and a top cover 6 which can be opened by means of a door 7.

At the top of stove 1 the front wall 4 has at least one opening 10, which can be provided in one or more of the stove walls, either the front wall 4 or the side 3a and 3b or rear walls 5. The or each opening 10 is preferably equipped with a protection grille 10a to allow an internal forced air circulation system, generically indicated by 8 and to be further described below, to take in ambient air. The forced air circulation system has at least one air outlet 11 at its base 2 at one or more walls of the stove. Each air outlet 11 is preferably equipped with a protection grille 11 a through which delivers air, possibly heated in the stove, into the environment or room in which the stove 1 is located.

If so desired, the rear panel or wall 5, at the base 2, can be formed with a further rear opening 12 for delivering processed forced air designed for connection to a duct leading to rooms other than that in which the stove 1 is installed.

Above opening 12, the rear panel 5 is formed with an opening 15 for combustion air intake. Of course, an opening 16 for communication with a fume exhaust flue is also provided.

As shown in Figures 1, 2 and 4, the forced air circulation system 8 includes a duct 17 through which the intake opening or openings 10 communicates with an electric intake fan 23 of suitable power rating.

In an intermediate position between the intake opening 10 and the electric fan 23, inside the duct 17, one or more ionisation devices 21 are installed, preferably each comprising a multiplicity of pairs of opposing points or needles 21 a, 21 b between which a voltage (e.g. 5 - 6 kV) is applied which has the effect of ionising the air passing through them.

In particular, the ionisation device 21 is advantageously powered by an electronic control unit 28, preferably including a board of 'vetronite', which is a material capable of withstanding temperatures of about 60°C. The board is equipped with a switching power circuit 28a, connected to a power source, e.g. to power supply mains (220V a.c.) via suitable power cables 29 (Figure 5). Between the switching power circuit 28a and the ionisation device 21 a voltage multiplier 28b is installed, which can comprise, e.g. five voltage doubler cells, and a current limiter 28c, e.g. a pair of resistors. The electronic unit 28 is connected to the ionisation device 21 through highly insulated cables 28d.

A control keyboard 30 is installed in such a ways as to be easily accessible from outside the body of stove 1 and has controls (keys or buttons) for switching the switching power circuit 28a on/off.

Advantageously, inside duct 17, preferably close to opening 10, a filter 20 of any suitable type can be installed for purifying the air flowing through opening 10. Such filter 20 is preferably equipped with clogging detecting means (not shown in the drawings and of any suitable type). In this case, unit 28 will also include an alarm circuit 28f connected at one end thereof to the clogging detecting means as generically shown with a cable 28e, and, at the other, to the keyboard 30 where a suitable warning device, e.g. a LED 30a, is located.

An electric fan 26 blowing on the board or unit 28 is preferably located at the intake opening 10 for the air to be treated to ensure an adequate flow of cooling air towards the said electronic board 28.

With specific reference to Figure 2, it will be noted that the flow of air entering through the intake grille 10a flows onto the ionisation device 21 after having been first filtered by the purifying filter 20, whereby the circulating air is both purified and ionised.

In practice and to enable easy access for maintenance, the board or unit 28 is mounted close to the intake opening 10 and the ionisation device 21 to which it is connected by cables 28d.

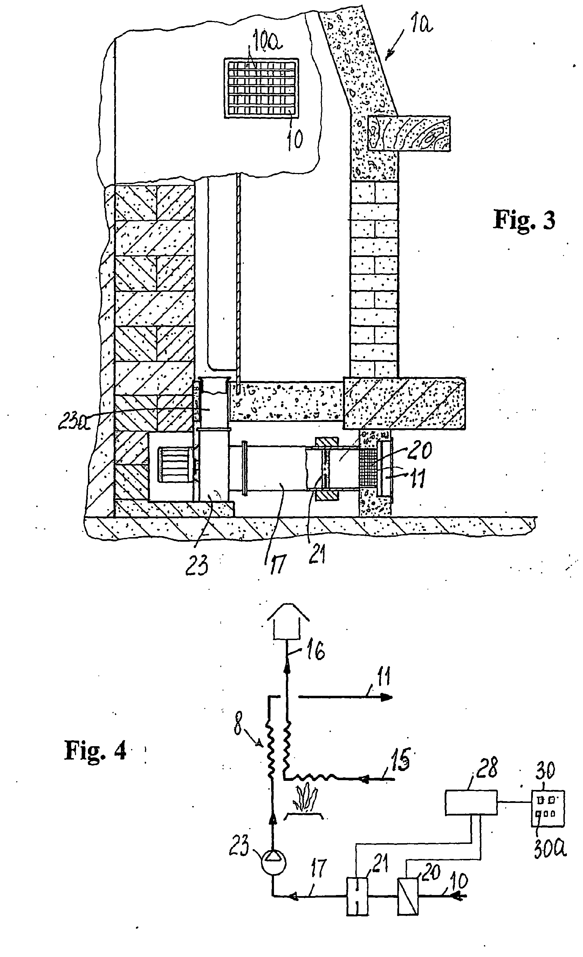

The ventilated fireplace 1 a shown in Figure 3 is equipped with a forced air circulation system 8 similarly to stove 1, and is provided with one or more intake openings 10 protected by a protection grille 10a, and with a duct 17 for communication with an electric intake fan 23 equipped with a duct 23a for delivering treated air issued from the delivery opening 11 also protected by a grille 11a,

Also in the case of a ventilated fireplace 1a there is provided inside duct 17 substantially at the intake opening or openings 10a an air purifying filter 20 of any suitable type, preferably equipped with clogging detector. Between the filter 20 and the electric fan 23, still inside the duct, an ionisation device 21 is provided.

The operation of the stove 1 or ventilated fireplace 1 a described above is quite simple and reliable.

The user who wishes to turn on the ionisation device 21, whether the stove 1 or fireplace 1 a is burning wood/pellets or the stove 1 or fireplace 1 a is off, e.g. during the warm period of the year, shall do so by operating a button on the keyboard 30 thus energizing the switching power circuit 28a. In this way air entering the intake opening or openings 10 is delivered by the electric fan 23 through filter 20 and onto the ionisation device 21, so as to be separated from its polluting particles, both due to the ionising effect of the ionisation device 21 and to the mechanical filtration action of filter 20. The air is subsequently delivered into the room via delivery opening 11 or, if provided, via ducts connected to delivery opening 12.

When the user wishes to switch off the switching power circuit 28a he will act upon the control keyboard 30, thus cutting off power to the ionisation device 21.

The user shall be notified of clogging of filter 20 by a luminous signal provided by the LED 30a on keyboard 30.

The invention described above is susceptible to numerous modifications and variations within the protection scope as defined in the claims.

In practice, the materials used as well as the dimensions of the assembly will vary according to requirements.

## Claims

1. Stove or ventilated fireplace including a forced air circulation system (8) and is **characterised in that** it comprises at least one air ionisation device (21).

2. Stove or ventilated fireplace according to claim 1, **characterised in that** the said ionisation device (21) comprises a plurality of opposing pairs of points or needles (21 a, 21 b).

3. Stove or ventilated fireplace according to claim 1 or 2, **characterised in that** the said ionisation device (21) includes an electronic control unit (28).

4. Stove or ventilated fireplace according to claim 3, **characterised in that** the said electronic control unit (28) comprises at least one switching power circuit (28a).

5. Stove or ventilated fireplace according to claim 3 or 4, **characterised in that** the said electronic control unit (28) comprises a voltage multiplier device (28b).

6. Stove or ventilated fireplace according to claim 3 or 4 or 5, **characterised in that** the said electronic control unit (28) comprises a current limiter equipment (28c).

7. Stove or ventilated fireplace according to claims 3 to 6, **characterised in that** the said electronic control board (28) comprises a control keyboard (30).

8. Stove or ventilated fireplace according to any previous claim, **characterised in that** it comprises an air purifying filter (20) between an intake opening (10) and said ionisation device (21).

9. Stove or ventilated fireplace according to claim 8, **characterised in that** the said air purifying filter (20) is equipped with clogging detecting means.

10. Stove or ventilated fireplace according to claim 9, **characterised in that** said clogging detecting means includes an alarm circuit (28f).

11. Stove or ventilated fireplace according to claim 10, when depending upon claim 7, **characterised in that** said keyboard (30) comprises a controlled warning means to indicate clogging of said filter (20),

12. Stove or ventilated fireplace according to any previous claim, **characterised in that** said ionisation device (21) is located substantially at an intake opening (10) of said forced air circulation system (8).

13. Stove or ventilated fireplace according to any previous claim, **characterised in that** it comprises an electric fan (26) to cool said electronic control unit (28).
